# EUROPEAN PATENT APPLICATION

(11) **EP 2 692 342 A1**
(43) Date of publication of application: **05.02.2014**
(21) Application number: 12178429.2
(22) Date of filing: 30.07.2012
(51) Int. Cl.: A61K 9/20, A61K 31/426

(54) **Process for the preparation of pharmaceutical compositions comprising febuxostat in the form of tablets**

(71) Applicant: Interquim, S.A., 08173 Sant Cugat del Vallés, Barcelona (ES)
(72) Inventor: Gibaja Ruiz, Manuel Francisco, 41004 Sevilla (ES); Muñoz Ruiz, Angel Jose, 41011 Sevilla (ES); Jurado Sanchez, Francisco, 08860 Castelldefels (ES); Martin Saiz, Pablo, 08172 Sant Cugat del Vallès (ES)
(74) Representative: ZBM Patents

(57) **Abstract**

The invention relates to a process for the preparation of pharmaceutical compositions comprising the steps of:
a.1) mixing or blending powders comprising febuxostat and at least one pharmaceutically acceptable excipient in solid form using convection mixers or blenders at least until a homogeneous mixture is obtained, optionally mixing or blending the previous mixture with further excipients in solid form; or alternatively
a.2) mixing or blending powders comprising febuxostat with at least one disintegrating agent in solid form until a homogeneous mixture is obtained, optionally mixing or blending the previous mixture with further excipients in solid form, wherein the w/w % of disintegrating agents is not lower than 8 % of the pharmaceutical composition; and
b) compressing the mixture of step (a) into tablets.

## Description

### Field of the invention

The invention relates to a process for the preparation of pharmaceutical compositions comprising febuxostat in the form of tablets, to the tablets obtainable by such process and to pharmaceutical mixtures comprising febuxostat.

### Background of the invention

Febuxostat is the International Non-Proprietary Name of 2-(3-cyano-4-isobutyloxyphenyl)-4-methyl-5-thiazolecarboxylic acid. Febuxostat, approved under the trade name Uloric^{®} in the USA and Adenuric^{®} in Europe, is a xanthine oxidase inhibitor indicated for the chronic management of hyperuricemia in patients with gout.

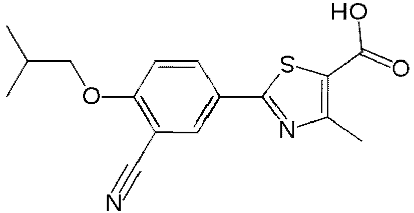

Febuxostat was first disclosed in W09209279 A1. A process for the manufacture of tablets comprising the wet granulation of products similar to febuxostat and excipients with a 20 % ethanol solution is described in W09209279 A1, but not specifically for febuxostat.

Similar tablets of febuxostat prepared by wet granulation with 20 % ethanol solution together with lactose, potato starch, polyvinyl pyrrolidone and magnesium stearate were disclosed in W09631211 A1.

Tablets comprising crystalline form A and other crystalline forms of febuxostat, prepared by wet granulation, lubrication and tabletting, were disclosed in W02003082279 A1. According to the description, crystalline form A of febuxostat shows a higher stability when prepared by this method and the dissolution profile does not change over time when using this form.

### Summary of the invention

In a first aspect the invention relates to a process for the preparation of pharmaceutical compositions comprising the steps of:
a) mixing or blending powders comprising febuxostat and at least one pharmaceutically acceptable excipient in solid form using convection mixers or blenders at least until a homogeneous mixture is obtained, optionally mixing or blending the previous mixture with further excipients in solid form; and
b) compressing the mixture of step (a) into tablets.

Surprisingly this process allows producing tablets showing faster dissolution. In general, processes of mixture or blending of active ingredients and excipients in solid form to be compressed are not deemed to be associated with differences in the dissolution profile of tablets thus obtained. In fact this effect was found by chance and, after that finding, tests were repeated because it was originally considered that the results were due to an experimental mistake. In order to produce tablets showing faster dissolution the person skilled in pharmaceutical technology would have typically resorted to reduce the mean particle size of the active ingredient, to use an amorphous form or a readily dissolvable crystalline form of the active ingredient or to use other processes such as wet granulation or melt granulation that are well-known to accelerate the dissolution of tablets.

In a second aspect the invention relates to a process for the preparation of pharmaceutical compositions comprising the steps of:
a) mixing or blending powders comprising febuxostat with at least one disintegrating agent in solid form until a homogeneous mixture is obtained, optionally mixing or blending the previous mixture with further excipients in solid form; and
b) compressing the mixture of step (a) into tablets,
wherein the w/w % of disintegrating agents is not lower than 8 % of the pharmaceutical composition.

This second aspect also provides an alternative solution to increase the speed of dissolution. Disintegrating agents are usually employed in the art in lower amounts, generally below 5 w/w %.

In a third aspect the invention relates to tablets obtainable by the processes according to any of the preceding aspects and embodiments thereof.

In a fourth aspect the invention relates to a mixture comprising febuxostat and at least one pharmaceutically acceptable excipient mixed or blended in solid form using convection mixers or blenders at least until a homogeneous mixture is obtained. These mixtures are useful intermediates for the processes and tablets of the invention.

### Definitions

In the present document the terms 'mixture' and 'blend' are considered to be synonymous to each other and will be used interchangeably unless otherwise stated. The same occurs with the related pairs of terms 'mixing' and 'blending'; and 'mixer' and 'blender'.

Blending and mixing is understood to be the reorientation of particles relative to one another in order to achieve uniformity. Blending and mixing processes can be operated under diffusion, convection and pneumatic mixing.

In diffusion mixing particles are reoriented in relation to one another when they are placed in random motion and interparticular friction is reduced as the result of bed expansion (usually within a rotating container); also known as tumble blending. Diffusion mixers are the most usual equipment employed to blend powders prior to compression. Turbula^{®} is an example of diffusion mixer.

In convection mixers or blenders particles are reoriented in relation to one another as a result of mechanical movement of mechanical means such as blades, paddles, screw, or plows.

In pneumatic mixing, particles are reoriented in relation to one another as a result of the expansion of a powder bed by gas.

Convection mixers or blenders include those that operate under the principles of convection mixing as described above. In particular convection mixers or blenders include those mentioned in the "Guidance for Industry SUPAC-IR/MR: Immediate release and Modified Release Solid Oral Dosage Forms" (Manufacturing Equipment Addendum U.S. Department of Health and Human Services Food and Drug Administration; Center for Drug Evaluation and Research (CDER); January 1999; CMC 9 Revision 1). This Guidance refers to the three types of operating principles for blending and mixing equipment discussed above.

Convection mixers or blenders include, but are not limited to, ribbon blenders, orbiting screw blenders, planetary blenders, Forberg blenders, horizontal double arm blenders, horizontal high intensity mixers, vertical high intensity mixers, diffusion mixers (tumble) with intensifier/agitator.

By the term "disintegrating agent" it is meant an agent which accelerates the disintegration of solid medicines. Disintegrating agents include cellulose or a cellulose derivative such as sodium or calcium carboxymethyl cellulose or crosslinked sodium carboxymethyl cellulose (croscarmellose sodium), crospovidone, pregelatinized starch, sodium carboxymethyl starch, alginic acid, sodium alginate, sodium starch glycolate or guar gum.

Dv50 denotes the diameter of the particles at which half of the total volume of the particles is below this figure. Dv50 can be determined by laser light diffraction according to the method described in the monograph 2.9.31 of the European Pharmacopeia, version 7.0.

Calcium stearate, glyceryl monostearate, magnesium stearate, stearic acid and talc are considered to be hydrophobic lubricants. In contrast, lubricants with a hydrophilic character include sodium stearyl fumarate, polyethylene glycol, sodium lauryl sulfate, sodium benzoate and those other lubricants with a solubility in water at 25 °C equal or higher than 1 g in 20 L.

By 'compressing the mixture of step (a) into tablets' it should be understood that if after the mixing or blending according to step (a), mixtures are submitted to processes in solid state such as processes to ensure the uniformity, sieving, milling or compactation, the mixtures thus obtained are still to be understood as the 'mixtures of step (a)'.

Regarding X-Ray diffraction data, peaks at 2θ angle (°) are to be understood as measured in a X-Ray diffractometer with Cu Kα radiation (λ=15418Å).

Febuxostat crystalline form A shows a X-Ray powder diffraction pattern comprising significant peaks at 2θ angle (°) values of about 6.62, 7.18, 12.80, 13.26, 16.48, 19.58, 21.92, 22.68, 25.84, 26.70, 29.16 and 36.70 as disclosed in WO9965885-A1.

Febuxostat crystalline form B shows a X-Ray powder diffraction pattern comprising significant peaks at 2θ angle (°) values of about 6.76, 8.08, 9.74, 11.50, 12.22, 13.56, 15.76, 16.20, 17.32, 19.38, 21.14, 21.56, 23.16, 24.78, 25.14, 25.72, 26.12, 26.68, 27.68 and 29.36 as disclosed in WO9965885-A1.

Febuxostat crystalline form C shows a X-Ray powder diffraction pattern comprising significant peaks at 2θ angle (°) values of about 6.62, 10.82, 13.36, 15.52, 16.74, 17.40, 18.00, 18.70, 20.16, 20.62, 21.90, 23.50, 24.78, 25.18, 34.08, 36.72 and 38.04 as disclosed in WO9965885-A1.

Febuxostat crystalline form D shows a X-Ray powder diffraction pattern comprising significant peaks at 2θ angle (°) values of about 8.32, 9.68, 12.92, 16.06, 17.34, 19.38, 21.56, 24.06, 26.00, 30.06, 33.60 and 40.34 as disclosed in WO9965885-A1.

Febuxostat crystalline form G shows a X-Ray powder diffraction pattern comprising significant peaks at 2θ angle (°) values of about 6.86, 8.36, 9.60, 11.76, 13.74, 14.60, 15.94, 16.74, 17.56, 20.00, 21.26, 23.72, 24.78, 25.14, 25.74, 26.06, 26.64, 27.92, 28.60, 29.66 and 29.98 as disclosed in WO9965885-A1.

Febuxostat amorphous form E shows a X-Ray powder diffraction pattern typical of an amorphous product and is described in WO9965885-A1.

Febuxostat crystalline form I shows a X-Ray powder diffraction pattern comprising significant peaks at 2θ angle (°) values of about 6.60, 7.20, 11.84, 12.82, 13.26, 16.46,17.82,19.04, 19.56, 21.94, 22.72, 23.80, 24.28, 24.68, 25.88, 26.68, 37.76 as disclosed in CN101139325-A.

Febuxostat crystalline form II shows a X-Ray powder diffraction pattern comprising significant peaks at 2θ angle (°) values of about 4.78, 6.82, 8.30, 9.58, 11.74, 13.74, 14.60, 15.92, 16.68, 17.56, 21.26, 23.64, 24.80, 25.20, 25.78, 26.08, 26.66, 28.66, 31.64 as disclosed in CN101139325-A.

Febuxostat crystalline form III shows a X-Ray powder diffraction pattern comprising significant peaks at 2θ angle (°) values of 5.8, 8.0, 12.8 and 25.9 as disclosed in CN101085761 A.

Febuxostat crystalline form H shows X-Ray powder diffraction patterns comprising significant peaks at 2θ angle (°) values of about 6.71, 7.19, 10.03, 11.10, 12.96 and 13.48 as disclosed in W008067773-A1.

Febuxostat crystalline form I shows X-Ray powder diffraction patterns comprising significant peaks at 2θ angle (°) values of about 3.28, 6.58, 12.7, 13.34, 19.97, 24.26 and 25.43 as disclosed in WO08067773-A1.

Febuxostat crystalline form J shows X-Ray powder diffraction patterns comprising significant peaks at 2θ angle (°) values of about 3.07, 12.25, 13.16, 25.21 and 26.86 as disclosed in WO08067773-A1.

It is well known in the art that peaks appearing in powder XRD of the same crystalline form could show variations of ±0.2° at 2θ angle values due to measurement inaccuracies of the powder X-Ray diffraction experiments.

The same effect of accelerating the dissolution of febuxostat in tablets is observed with all crystalline forms tested.

Particle size of all crystalline forms can be adjusted according to well-known method for the person skilled in the art such as micronization.

All mixtures should be prepared until they are homogeneous. To ascertain homogeneity of the powder mixtures it is particularly suitable to use the criteria established in the 'Guidance for Industry: Powder Blends and Finished Dosage Units — Stratified In-Process Dosage Unit Sampling and Assessment', DRAFT GUIDANCE, October 2003, U.S. Department of Health and Human Services; Food and Drug Administration; Center for Drug Evaluation and Research (CDER) and the revised attachments of the above Guidance of November 2003. In the cases where powder blend does not meet the homogeneity criteria described in the cited guidance, but the Final Dosage Unit comprising said powder blend meets the Uniformity of Dosage Units described in the USP <905> (United States Pharmacopeia 34 or USP 34), it can be considered that the blend was homogeneous itself even if it cannot be demonstrated by analytical data of blend samples. That could happen due to sampling errors or inappropriate processing of samples. Depending on the equipment to be used for mixing or blending, the determination of the conditions to be used to achieve homogeneous mixtures such as the time of mixture and speed of the impeller could easily be found by the person skilled in the art without undue burden.

### Detailed description of the invention

The following is a numbered list of embodiments according to the invention.
Embodiment 1. A process for the preparation of pharmaceutical compositions comprising the steps of:
   a) mixing or blending powders comprising febuxostat and at least one pharmaceutically acceptable excipient in solid form using convection mixers or blenders at least until a homogeneous mixture is obtained, optionally mixing or blending the previous mixture with further excipients in solid form; and
   b) compressing the mixture of step (a) into tablets.
Embodiment 2. The process according to the preceding embodiment, wherein the convection mixer or blender is selected from the list consisting of ribbon blenders, orbiting screw blenders, planetary blenders, Forberg blenders, horizontal double arm blenders, horizontal high intensity mixers, vertical high intensity mixers, diffusion mixers (tumble) with intensifier/agitator or combinations thereof.
Embodiment 3. The process according to any of the preceding embodiments, wherein said pharmaceutically acceptable excipient comprises at least one disintegrating agent.
Embodiment 4. The process according to the preceding embodiment, wherein the w/w % of disintegrating agents is not lower than 8 % of the pharmaceutical composition.
   Using such proportion of disintegrating agents improves the speed of dissolution of tablets as can be seen in the examples.
Embodiment 5. A process for the preparation of pharmaceutical compositions comprising the steps of:
   a) mixing or blending powders comprising febuxostat with at least one disintegrating agent in solid form until a homogeneous mixture is obtained, optionally mixing or blending the previous mixture with further excipients in solid form; and
   b) compressing the mixture of step (a) into tablets, wherein the w/w % of disintegrating agents is not lower than 8 % of the pharmaceutical composition.
Embodiment 6. The process according to any of the embodiments 4 or 5, wherein the w/w % of disintegrating agents is not lower than 10 %.
Embodiment 7. The process according to the preceding embodiment, wherein the w/w % of disintegrating agents is not lower than 10 % and not higher than 20 %.
Embodiment 8. The process according to the preceding embodiment, wherein the w/w % of disintegrating agents is not lower than 12 % and not higher than 18 %, preferably 15 %.
Embodiment 9. The process according to any of the embodiments 3 to 8, wherein at least one disintegrating agent is selected from the list consisting of sodium croscarmellose, crospovidone or combinations thereof.
Embodiment 10. The process according to the preceding embodiment, wherein at least one disintegrating agent is crospovidone.
   Crospovidone proved to be the best performing disintegrating agents in terms of accelerating the dissolution of febuxostat from tablets as shown in the examples.
Embodiment 11. The process according to any of the preceding embodiments, wherein febuxostat has a Dv50 of no less than 1 µm and no more than 10 µm, preferably no less than 2 µm and no more than 8 µm, more preferably no less than 2 µm and no more than 6 µm.
Embodiment 12. The process according to any of the preceding embodiments, wherein febuxostat comprises febuxostat crystalline form A that shows a X-Ray powder diffraction pattern comprising significant peaks at 2θ angle (°) values of about 6.62, 7.18, 12.80, 13.26, 16.48, 19.58, 21.92, 22.68, 25.84, 26.70, 29.16 and 36.70 ±0.2°;
   or febuxostat crystalline form B that shows a X-Ray powder diffraction pattern comprising significant peaks at 2θ angle (°) values of about 6.76, 8.08, 9.74, 11.50, 12.22, 13.56, 15.76, 16.20, 17.32, 19.38, 21.14, 21.56, 23.16, 24.78, 25.14, 25.72, 26.12, 26.68, 27.68 and 29.36 ±0.2°;
   or febuxostat crystalline form C that shows a X-Ray powder diffraction pattern comprising significant peaks at 2θ angle (°) values of about 6.62, 10.82, 13.36, 15.52, 16.74, 17.40, 18.00, 18.70, 20.16, 20.62, 21.90, 23.50, 24.78, 25.18, 34.08, 36.72 and 38.04 ±0.2°;
   or febuxostat crystalline form D that shows a X-Ray powder diffraction pattern comprising significant peaks at 2θ angle (°) values of about 8.32, 9.68, 12.92, 16.06, 17.34, 19.38, 21.56, 24.06, 26.00, 30.06, 33.60 and 40.34 ±0.2°;
   or febuxostat crystalline form G that shows a X-Ray powder diffraction pattern comprising significant peaks at 2θ angle (°) values of about 6.86, 8.36, 9.60, 11.76, 13.74, 14.60, 15.94, 16.74, 17.56, 20.00, 21.26, 23.72, 24.78, 25.14, 25.74, 26.06, 26.64, 27.92, 28.60, 29.66, and 29.98 ±0.2°;
   or febuxostat amorphous form E that shows a X-Ray powder diffraction pattern typical of an amorphous product and is described in WO9965885-A1;
   or febuxostat crystalline form I that shows a X-Ray powder diffraction pattern comprising significant peaks at 2θ angle (°) values of about 6.60, 7.20, 11.84, 12.82, 13.26, 16.46,17.82,19.04, 19.56, 21.94, 22.72, 23.80, 24.28, 24.68, 25.88, 26.68, 37.76 ±0.2°;
   or febuxostat crystalline form II that shows a X-Ray powder diffraction pattern comprising significant peaks at 2θ angle (°) values of about 4.78, 6.82, 8.30, 9.58, 11.74, 13.74, 14.60, 15.92, 16.68, 17.56, 21.26, 23.64, 24.80, 25.20, 25.78, 26.08, 26.66, 28.66, 31.64 ±0.2°;
   or crystalline form III of febuxostat that shows a X-Ray powder diffraction pattern comprising peaks at 2θ angle (°) values of about 5.8, 8.0, 12.8 and 25.9 ±0.2°;
   or febuxostat crystalline forms H that shows a X-Ray powder diffraction pattern comprising significant peaks at 2θ angle (°) values of about 6.71, 7.19, 10.03, 11.10, 12.96 and 13.48 ±0.2°;
   or febuxostat crystalline forms I that shows a X-Ray powder diffraction pattern comprising significant peaks at 2θ angle (°) values of about 3.28, 6.58, 12.7, 13.34, 19.97, 24.26 and 25.43 ±0.2°;
   or febuxostat crystalline forms J that shows a X-Ray powder diffraction pattern comprising significant peaks at 2θ angle (°) values of about 3.07, 12.25, 13.16, 25.21 and 26.86 ±0.2°;
   all of them measured in a X-Ray diffractometer with Cu Kα radiation (λ=1.5418Å).
Embodiment 13. The process according to any of the preceding embodiments, wherein at least one lubricant with hydrophilic character is used, preferably sodium stearyl fumarate.
Embodiment 14. The process according to any of the preceding embodiments, wherein tablets are coated.
Embodiment 15. The process according to any of the preceding embodiments not comprising any wet granulation step.
Embodiment 16. The process according to any of the embodiments 1 to 14 not comprising any dry granulation step.
Embodiment 17. The process according to any of the preceding embodiments not comprising any granulation step.
   The absence of granulation steps simplifies the process as additional steps are not required, which bears some cost reduction. Additionally, wet granulation processes can encompass changes in the crystalline form of febuxostat.
Embodiment 18. Tablets obtainable by the processes according to any of the preceding embodiments.
Embodiment 19. A mixture comprising febuxostat and at least one pharmaceutically acceptable excipient mixed or blended in solid form using convection mixers or blenders at least until a homogeneous mixture is obtained.

Example 1 compares two batches manufactured with w/w 5% croscarmellose sodium, a disintegrant, mixed using a convection mixer (1.a) and a diffusion mixer (1.b). The results obtained show that convection mixing provides faster dissolution at 15 minutes and less punch sticking.

Example 2 compares two batches manufactured with w/w 15% croscarmellose sodium, a disintegrant, mixed using a convection mixer (2.a) and a diffusion mixer (2.b). The results obtained show that convection mixing provides faster dissolution at 15 minutes and less punch sticking.

Example 3 compares two batches manufactured with w/w 15% crospovidone, a different disintegrant, mixed using a convection mixer (3.a) and a diffusion mixer (3.b). The results obtained show that convection mixing provides faster dissolution at 15 minutes and less punch sticking.

The three examples above show that convection mixing provides faster dissolution (greater amount dissolved at 15 minutes) and improved processing for febuxostat blends.

When a blend with w/w 15% disintegrant is mixed using convection mixing, the dissolution at 15 minutes is faster than that of a blend containing only w/w 5% disintegrant (batch 1.a vs 2.a). When blends with w/w 15% crospovidone are mixed by convection mixing, the dissolution at 15 minutes is faster than that of the equivalent blend containing 15% croscarmellose sodium (batch 2.b vs 3.a).

Example 4 shows that in blends having 10-20 w/w % crospovidone prepared by convection mixing, specially w/w 15 %, and including sodium stearyl fumarate as lubricant the amount dissolved at 15 minutes is high and that if w/w % crospovidone is 15 or 20 %, punch sticking is not observed.

Examples 4-6 show that the conclusions reached using small scale equipment (electric coffee grinder used as convection mixer) hold for laboratory-scale equipment (high-shear mixer-granulator Zanchetta Rotolab^{®}) and pilot-scale equipment (GEI-Collette).

As seen in the example 6, after obtaining a homogeneous blend, the acceleration of the dissolution rate of tablets is achieved independently of the blending time.

In general, tablets comprising more than 8 % of disintegrating agent show a significant amount dissolved at 15 minutes under the conditions described above.

The fact that the processes according to the invention can be performed in solid state minimizes the likelihood of polymorphic conversion of the active ingredient, febuxostat, during the manufacturing of tablets. This results in a more robust process for manufacturing pharmaceutical compositions.

### Examples

The following examples are not limitative in any way. In examples 1-5 tablets comprising 120 mg of febuxostat were prepared; while in example 6 tablets comprise 80 mg of febuxostat. Using the same procedure, tablets comprising other febuxostat strengths, such as 40, 80 and 120 mg/tablet, can be prepared.

Lactose DCL15 and Lactose GranuLac^{®} 70 were used as lactose monohydrate, Avicel^{®} Ph102, Vivapur^{®} 112 and Vivapur^{®} XLM 200 were used as microcrystalline cellulose, AC-DI-SOL^{®} was used as croscarmellose sodium, Kollidon^{®} CL was used as crospovidone source. Silicon dioxide used in the examples was colloidal silicon dioxide available as Aerosil^{®} 200. The person skilled in the art would easily recognize other suitable brands of excipients.

Unless otherwise stated, percentages (%) are weigh/weigh (w/w) percentages referred to the (total) weight of the pharmaceutical dosage form.

Febuxostat was prepared according to the process disclosed in W02012007487 A1.

Electric coffee grinder Fagor^{®} ML-150 V having blades rotating at high speed was used in some of the examples below as low-scale preliminary testing equipment for convection mixers. The results obtained with this equipment were later on confirmed on conventional manufacturing equipment as shown in examples 5 and 6.

Punch sticking was assessed visually based on a scale wherein '0' means 'no sticking','0.5' means 'moderate sticking' and '1' means 'significant sticking'.

Q15min % is the amount of febuxostat expressed as a % dissolved in 15 minutes of the total dose at pH=6.8 using a phosphate buffer when tablets are tested at 50 rpm, in a volume of 900mL, using UV detector at 315nm in a dissolution USP Apparatus 2.

### Example 1

### 1.a

According to the formula shown below, febuxostat and crosscarmellose sodium were blended in an electric coffee grinder having blades rotating at high speed for 180 seconds. The mixture was then blended in a Turbula^{®} T2F mixer with the remaining excipients (except for magnesium stearate) for 15 minutes at 34 rpm. Magnesium stearate was added to the blend and mixed for 5 minutes at 34 rpm in the same equipment (Turbula^{®} T2F). A homogeneous mixture was obtained. The bend was compressed into tablets in a tabletting machine.

| **Example 1.a** | **%** | **g/batch** |
|---|---|---|
| Febuxostat | 15.89 | 18.000 |
| Lactose monohydrate | 15.00 | 16.988 |
| Microcrystalline cellulose | 62.61 | 70.901 |
| Croscarmellose sodium | 5.00 | 5.663 |
| Silicon dioxide | 1.00 | 1.133 |
| Magnesium stearate | 0.50 | 0.566 |
| **TOTAL** | **100** | **113.250** |

### 1.b

According to the formula of Example 1.a, febuxostat and all the excipients (except for magnesium stearate) were blended in a Turbula^{®} T2F mixer with for 15 minutes at 34 rpm. Magnesium stearate was added to the blend and mixed for 5 minutes at 34 rpm in the same equipment (Turbula^{®} T2F). A homogeneous mixture was obtained. The bend was compressed into tablets in a tabletting machine.

### Example 2

### 2.a

According to the formula shown below, febuxostat and crosscarmellose sodium were blended in an electric coffee grinder having blades rotating at high speed for 180 seconds. The mixture was then blended in a Turbula^{®} T2F mixer with the remaining excipients (except for magnesium stearate) for 15 minutes at 34 rpm. Magnesium stearate was added to the blend and mixed for 5 minutes at 34 rpm in the same equipment (Turbula^{®} T2F). A homogeneous mixture was obtained. The bend was compressed into tablets in a tabletting machine.

| **Example 2.a** | **%** | **g/batch** |
|---|---|---|
| Febuxostat | 15.89 | 18.000 |
| Lactose monohydrate | 15.00 | 16.988 |
| Microcrystalline cellulose | 52.61 | 59.576 |
| Croscarmellose sodium | 15.00 | 16.988 |
| Silicon dioxide | 1.00 | 1.133 |
| Magnesium stearate | 0.50 | 0.566 |
| **TOTAL** | **100** | **113.250** |

### 2.b

According to the formula of Example 2.a, febuxostat and crosscarmellose sodium were blended in a Turbula^{®} T2F mixer with the remaining excipients (except for magnesium stearate) for 15 minutes at 34 rpm. Magnesium stearate was added to the blend and mixed for 5 minutes at 34 rpm in the same equipment (Turbula^{®} T2F). A homogeneous mixture was obtained. The bend was compressed into tablets in a tabletting machine.

### Example 3

### 3.a

According to the formula shown below, febuxostat and crospovidone were blended in an electric coffee grinder having blades rotating at high speed for 180 seconds. The mixture was then blended in a Turbula^{®} T2F mixer with the remaining excipients (except for magnesium stearate) for 15 minutes at 34 rpm. Magnesium stearate was added to the blend and mixed for 5 minutes at 34 rpm in the same equipment (Turbula^{®} T2F). A homogeneous mixture was obtained. The bend was compressed into tablets in a tabletting machine.

| **Example 3.a** | **%** | **g/batch** |
|---|---|---|
| Febuxostat | 15.89 | 18.000 |
| Lactose monohydrate | 15.00 | 16.988 |
| Microcrystalline cellulose | 52.61 | 59.576 |
| Crospovidone | 15.00 | 16.988 |
| Silicon dioxide | 1.00 | 1.133 |
| Magnesium stearate | 0.50 | 0.566 |
| **TOTAL** | **100** | **113.250** |

### 3.b

According to the formula of Example 3.a, febuxostat and crospovidone were blended in a Turbula^{®} T2F mixer with the remaining excipients (except for magnesium stearate) for 15 minutes at 34 rpm. Magnesium stearate was added to the blend and mixed for 5 minutes at 34 rpm in the same equipment (Turbula^{®} T2F). A homogeneous mixture was obtained. The bend was compressed into tablets in a tabletting machine.

### Example 4

### 4.a1 crospovidone 10 %

According to the formula of Example 4.a1, lactose, cellulose, crospovidone and silicon dioxide were blended in a Zanchetta Rotolab^{®} high-shear mixer-granulator for 3 minutes at 600 rpm. Febuxostat was added and mixed for 5 additional minutes at 600 rpm in the same equipment. Sodium stearyl fumarate was added and mixed for 5 additional minutes at 600 rpm in the same equipment. A homogeneous mixture was obtained. The bend was compressed into tablets in a tabletting machine.

| **Example 4.a1** | **%** | **g/batch** |
|---|---|---|
| Febuxostat | 16.00 | 60.000 |
| Lactose monohydrate | 15.30 | 57.375 |
| Microcrystalline cellulose | 54.70 | 205.125 |
| Crospovidone | 10.00 | 37.500 |
| Silicon dioxide | 1.00 | 3.750 |
| Sodium stearyl fumarate | 3.00 | 11.250 |
| **TOTAL** | 100 | 375.000 |

### 4.a2 crospovidone 15 %

According to the formula of Example 4.a2, lactose, cellulose, crospovidone and silicon dioxide were blended in a Zanchetta Rotolab^{®} high-shear mixer-granulator for 3 minutes at 600 rpm. Febuxostat was added and mixed for 5 additional minutes at 600 rpm in the same equipment. Sodium stearyl fumarate was added and mixed for 5 additional minutes at 600 rpm in the same equipment. A homogeneous mixture was obtained. The bend was compressed into tablets in a tabletting machine.

| **Example 4.a2** | **%** | **g/batch** |
|---|---|---|
| Febuxostat | 16.00 | 90.000 |
| Lactose monohydrate | 15.30 | 86.063 |
| Microcrystalline cellulose | 49.70 | 279.563 |
| Crospovidone | 15.00 | 84.375 |
| Silicon dioxide | 1.00 | 5.625 |
| Sodium stearyl fumarate | 3.00 | 16.875 |
| **TOTAL** | 100 | 562.500 |

### 4.a3 crospovidone 20 %

According to the formula of Example 4.a3, lactose, cellulose, crospovidone and silicon dioxide were blended in a Zanchetta Rotolab^{®} high-shear mixer-granulator for 3 minutes at 600 rpm. Febuxostat was added and mixed for 5 additional minutes at 600 rpm in the same equipment. Sodium stearyl fumarate was added and mixed for 5 additional minutes at 600 rpm in the same equipment. A homogeneous mixture was obtained. The bend was compressed into tablets in a tabletting machine.

| **Example 4.a3** | **%** | **g/batch** |
|---|---|---|
| Febuxostat | 16.00 | 60.000 |
| Lactose monohydrate | 15.30 | 57.375 |
| Microcrystalline cellulose | 44.70 | 167.625 |
| Crospovidone | 20.00 | 75.000 |
| Silicon dioxide | 1.00 | 3.750 |
| Sodium stearyl fumarate | 3.00 | 11.250 |
| **TOTAL** | 100 | 375.000 |

### Example 5

According to the formula shown below, lactose, microcrystalline cellulose, crospovidone and silicon dioxide were blended in a high-shear mixer-granulator Zanchetta Rotolab^{®} (a convection mixer) for 3 minutes with an impeller speed of 600 rpm. Febuxostat was added to the blend and mixed for another 5 minutes at an impeller speed of 600 rpm. The blend obtained was screened through a 500 µm mesh and lubricated by adding magnesium stearate and mixing for 5 minutes in a Turbula^{®} T2F mixer at 34 rpm. A homogeneous mixture was obtained. The bend was compressed into tablets in a tabletting machine.

| **Example 5** | **%** | **g/batch** |
|---|---|---|
| Febuxostat | 15.80 | 60.000 |
| Lactose monohydrate | 15.00 | 56.625 |
| Microcrystalline cellulose | 52.61 | 198.588 |
| Crospovidone | 15.00 | 56.625 |
| Silicon dioxide | 1.00 | 3.775 |
| Magnesium stearate | 0.50 | 1.888 |
| **TOTAL** | **100** | **377.500** |

**Summary of examples 1-5 and results**

| **Example** | **1.a %** | **2.a %** | **3.a %** | **4.a1 %** | **4.a2 %** | **4.a3 %** | **5%** |
|---|---|---|---|---|---|---|---|
| Febuxostat | 15.89 | 15.89 | 15.89 | 16.00 | 16.00 | 16.00 | 15.80 |
| Lactose monohydrate | 15.00 | 15.00 | 15.00 | 15.30 | 15.30 | 15.30 | 15.00 |
| Microcrystalline cellulose | 62.61 | 52.61 | 52.61 | 54.70 | 49.70 | 44.70 | 52.61 |
| Croscarmellose sodium | 5.00 | 15.00 | - | - | - | - | - |
| Crospovidone | - | - | 15.00 | 10.00 | 15.00 | 20.00 | 15.00 |
| Silicon dioxide | 1.00 | 1.00 | 1.00 | 1.00 | 1.00 | 1.00 | 1.00 |
| Magnesium stearate | 0.50 | 0.50 | 0.50 | | - | - | 0.50 |
| Sodium stearyl fumarate | - | - | - | 3.00 | 3.00 | 3.00 | - |
| **TOTAL** | **100** | **100** | **100** | **100** | **100** | **100** | **100** |

| **Example** | **Blending** | **Disintegrant** | **% Disintegrant** | **Q15min (pH 6.8) %** | **SD Q15min (pH 6.8)** | **Punch Sticking** |
|---|---|---|---|---|---|---|
| 1.a | Convection | Croscarmellose | 5.00 | 41.70 | 2.7 | 0.5 |
| 1.b | Diffusion | Croscarmellose | 5.00 | 33.00 | 2.2 | 1 |
| 2.a | Convection | Croscarmellose | 15.00 | 71.00 | 5.9 | 0.5 |
| 2.b | Diffusion | Croscarmellose | 15.00 | 32.50 | 1.3 | 1 |
| 3.a | Convection | Crospovidone | 15.00 | 92.30 | 1.3 | 0.5 |
| 3.b | Diffusion | Crospovidone | 15.00 | 43.10 | 3.3 | 1 |
| 4.a1 | Convection | Crospovidone | 10.00 | 84.19 | 1.0 | 1 |
| 4.a2 | Convection | Crospovidone | 15.00 | 86.24 | 1.3 | 0 |
| 4.a3 | Convection | Crospovidone | 20.00 | 70.66 | 6.9 | 0 |
| 5 | Convection | Crospovidone | 15.00 | 93.88 | 1.7 | 0 |

### Example 6

Tablets were prepared according to the formula shown in the table below:

| **Example 6** | **%** | **g/batch** |
|---|---|---|
| Febuxostat | 16.00 | 800 |
| Lactose monohydrate | 15.30 | 765 |
| Microcrystalline cellulose | 49.70 | 2,485 |
| Crospovidone | 15.00 | 750 |
| Silicon dioxide | 1.00 | 50 |
| Sodium stearyl fumarate | 3.00 | 150 |
| **TOTAL** | **100** | **5,000** |

following the next steps:
i) lactose, microcrystalline cellulose, crospovidone and anhydrous colloidal silica (silicon dioxide) were blended in a convection blender (GEI-Collette, a type of vertical high intensity mixer) having blades rotating at high speed (200 rpm) for 4 minutes;
ii) mixtures of step (i) were then blended with febuxostat in the same convection blender (under the same conditions) using different mixing times as detailed in the table below;
iii) the lubricant, sodium stearyl fumarate, was added to the mixture of step (ii) and mixed (under the same conditions) using different mixing times as detailed in the table below; and
iv) the homogeneous blends obtained in step (iii) were tabletted in a tablet press machine. Tablets having different values of hardness were obtained as detailed in the table below.

| **Example** | **Blending** | **Mixing time of steps (i)+(ii)+(iii) in minutes** | **Hardness** | **Q15min (pH 6.8) %** | **SD Q15min (pH 6.8)** |
|---|---|---|---|---|---|
| 6.a1 | Convection | 4'+7'+7' | 90 N | 90.02 | 2.24 |
| | | | 120 N | 88.49 | 11.51 |
| 6.a2 | Convection | 4'+10'+10' | 90 N | 93.33 | 1.47 |
| | | | 120 N | 95.61 | 1.82 |
| 6.a3 | Convection | 4'+13'+13' | 90 N | 95.32 | 0.31 |
| | | | 115 N | 98.20 | 4.48 |
| | | | 150 N | 97.63 | 4.79 |
| 6.a4 | Convection | 4'+20'+20' | 120 N | 98.08 | 2.10 |
| | | | 150 N | 96.49 | 3.71 |

No punch sticking was observed in any of the tests of example 6.

## Claims

1. A process for the preparation of pharmaceutical compositions comprising the steps of:
a) mixing or blending powders comprising febuxostat and at least one pharmaceutically acceptable excipient in solid form using convection mixers or blenders at least until a homogeneous mixture is obtained, optionally mixing or blending the previous mixture with further excipients in solid form; and
b) compressing the mixture of step (a) into tablets.

2. The process according to the preceding claim, wherein the convection mixer or blender is selected from the list consisting of ribbon blenders, orbiting screw blenders, planetary blenders, Forberg blenders, horizontal double arm blenders, horizontal high intensity mixers, vertical high intensity mixers, diffusion mixers (tumble) with intensifier/agitator or combinations thereof.

3. The process according to any of the preceding claims, wherein said pharmaceutically acceptable excipient comprises at least one disintegrating agent.

4. The process according to the preceding claim, wherein the w/w % of disintegrating agents is not lower than 8 % of the pharmaceutical composition.

5. A process for the preparation of pharmaceutical compositions comprising the steps of:
a) mixing or blending powders comprising febuxostat with at least one disintegrating agent in solid form until a homogeneous mixture is obtained, optionally mixing or blending the previous mixture with further excipients in solid form; and
b) compressing the mixture of step (a) into tablets, wherein the w/w % of disintegrating agents is not lower than 8 % of the pharmaceutical composition.

6. The process according to any of the claims 4 or 5, wherein the w/w % of disintegrating agents is not lower than 10 %.

7. The process according to the preceding claim, wherein the w/w % of disintegrating agents is not lower than 10 % and not higher than 20 %.

8. The process according to the preceding claim, wherein the w/w % of disintegrating agents is not lower than 12 % and not higher than 18 %.

9. The process according to any of the claims 3 to 8, wherein at least one disintegrating agent is selected from the list consisting of sodium croscarmellose, crospovidone or combinations thereof.

10. The process according to the preceding claim, wherein at least one disintegrating agent is crospovidone.

11. The process according to any of the preceding claims, wherein febuxostat has a Dv50 of no less than 1 µm and no more than 10 µm, preferably no less than 2 µm and no more than 8 µm, more preferably no less than 2 µm and no more than 6 µm.

12. The process according to any of the preceding claims, wherein at least one lubricant with hydrophilic character is used, preferably sodium stearyl fumarate.

13. The process according to any of the preceding claims, wherein tablets are coated.

14. Tablets obtainable by the processes according to any of the preceding claims.

15. A mixture comprising febuxostat and at least one pharmaceutically acceptable excipient mixed or blended in solid form using convection mixers or blenders at least until a homogeneous mixture is obtained.
